# EUROPÄISCHE PATENTSCHRIFT

(11) **EP 1 140 135 B1**
(45) Veröffentlichungstag und Bekanntmachung des Hinweises auf die Patenterteilung: **17.09.2003**
(21) Anmeldenummer: 99964670.6
(22) Anmeldetag: 23.12.1999
(51) Int. Cl.: A61K 38/13, A61K 9/48, A61K 47/10, A61K 9/08

(54) **CYCLOSPORIN-LÖSUNG**
CYCLOSPORIN SOLUTION
SOLUTION DE CYCLOSPORINE

(30) Priorität: 23.12.1998 DE 19859910
(43) Veröffentlichungstag der Anmeldung: 10.10.2001
(73) Patentinhaber: ratiopharm GmbH, 89070 Ulm (DE)
(72) Erfinder: FISCHER, Wilfried, D-85579 Neubiberg (DE)
(74) Vertreter: Lederer, Franz, Dr.
(86) Internationale Anmeldenummer: EP9910358
(87) Internationale Veröffentlichungsnummer: WO00038702

(56) Entgegenhaltungen:
- EP-A- 0 711 550
- WO-A-98/40094
- GB-A- 2 228 198
- US-A- 4 996 193
- US-A- 5 798 333

## Beschreibung

Die vorliegende Erfindung betrifft eine Cyclosporin-Lösung.

Cyclosporine sind eine bekannte Gruppe cyclischer Undekapeptide. Cyclosporin A (C₆₂H₁₁₁N₁₁O₁₂, Molekulargewicht 1202) findet Anwendung als immunsupprimierendes Arzneimittel zur Behandlung von Gewebeabstoßungsreaktionen oder überschießenden Immunreaktionen des Körpers und ist beispielsweise als Sandimmun® und Neoral® im Handel erhältlich. Neben Cyclosporin A sind eine Reihe von Nebenmetaboliten bekannt (Cyclosporine B-Z), die strukturell und zum Teil auch wirkungsmäßig eine nahe Verwandtschaft zu Cyclosporin A aufweisen.

Der internationale Freiname für ein zur Immunsuppression verwendetes Cyclosporin ist Ciclosporin.

Es ist darüber hinaus bekannt, daß Cyclosporin A in Wasser sehr schlecht löslich ist. Hieraus ergibt sich die Problematik der Formulierung von gut und schnell absorbierbaren pharmazeutischen Cyclosporin A-Zubereitungen, da die schnelle und vollständige oder nahezu vollständige Absoption des Wirkstoffs eine unabdingbare Voraussetzung für eine zuverlässige Wirksamkeit bei den lebenswichtigen Indikationen wie der Unterdrückung der Gewebeabstoßung nach Organtransplantationen ist. Im Stand der Technik wurden zahlreiche Versuche unternommen, Cyclosporin A in einer gut absorbierbaren Formulierung zur Verfügung zu stellen. Aufgrund der großen Lipophilie des Cyclosporin A wurden pharmazeutische Zusammensetzungen mit üblichen festen und flüssigen pharmazeutischen Trägerstoffen formuliert, die jedoch oft Nachteile, wie eine unzureichende Resorption (Cavanak und Sucker, Formulation of Dosage Forms, Prog. Allergy, 38, 65-72 (1986)), eine schlechte Verträglichkeit oder physikalische Instabilitäten wie ein Auskristallisieren des Wirkstoffes aufwiesen. Auch erwies es sich als nachteilig, daß die Löslichkeit des Wirkstoffes in der Zubereitung vielfach gering ist (ca. 3%), was bei einer täglichen Dosis von bis zu 1 g Cyclosporin A eine Einnahmemenge von bis zu 30 g der Formulierung bedeutet.

Das Patent DE 29 07 460 offenbart zur Lagerungs- und Resorptionsverbesserung von Cyclosporin A den Einsatz eines Trägerstoffs aus einem Polyalkylenglycol-Triglycerid, einem Fettsäuretriglycerid und einem Mono- oder Diglycerid. Die Formulierung findet als Trinklösung, Injektionslösung oder Kapselinhalt Verwendung. Zur Löslichkeitsförderung kann Ethanol zugesetzt werden. Eine solche Lösung wird relativ gut resorbiert, jedoch mit dem Nachteil, daß der Blutspiegel sehr stark variieren kann und von der Nahrungsmittelaufnahme abhängig ist.

Eine verbesserte Formulierung wird in DE 39 30 928 als sogenanntes Mikroemulsionsvorkonzentrat beschrieben, das aus einer hydrophilen Phase, einer lipophilen Phase und einem Emulagator besteht. Die hydrophile Komponente kann ein C₁₋₅-Alkyl- oder Tetrahydrofurfuryldiether oder ein Partialether niedermolekularer Mono- oder Polyoxyalkandiole oder 1,2-Propylenglycol sein. Die lipophile Komponente kann ein mittelkettiges Triglycerid sein. Als Emulagtor wird z.B. ein polyethoxyliertes Pflanzenöl vorgesehen.

In einer vergleichenden Absorptionsstudie an Beagle Hunden konnte eine 49%-ige Verbesserung der Absorption im Vergleich zu der in dem DE 29 07 460 offenbarten Formulierung gefunden werden.

Die DE 195 21 974 beschreibt eine Lösung von Cyclosporin A in einer Mischung aus einem emulgierenden Vitamin E-Derivat, einem weiteren Emulgator, wie einem Polyoxyethylen-Pflanzenölester und Ethanol. Die Formulierung zeigt einen zur Formulierung der DE 39 30 928 vergleichbaren Blutspiegelverlauf in Beagle Hunden.

Die WO 97/35603 beschreibt eine Mikrodispersion, umfassend amorphes Cyclosporin A, niedere Alkanole und Polyoxyalkylenemulgatoren als Co-Lösungsmittel.

Die WO 97/07787 offenbart eine Cyclosporinformulierung, die ein Alkanollösungsmittel mit 2 bis 3 Kohlenstoffatomen und einen Emulgator umfaßt, ausgewählt aus Polyoxyethylenalkoholen und Fettsäuremonoestern von ethoxylierten C₄₋₆-Polyolen.

Das Dokument EP-A-711550 offenbart eine Cyclosporinweichkapselzusammensetzung, entahltend Dimethylisosorbid als Cooberflächenaktives Mittel, eine Komponente oder ein Gemisch aus zwei Komponenten, ausgewählt aus einer veresterten Verbindung von Fettsäure und primären Alkohol, einem Fettsäuretriglycerid mittlerer Kettenlänge und einem Fettsäuremonoglycerid als Ölkomponenten und einem oberflächenaktiven Mittel mit einem HLB Wert von 10 bis 17.

Das Dokument WO-A-9840094 offenbart eine pharmazeutische Zusammnesetzung enthaltend Cyclosporon, eine hydrophile Phase, und ein oberflächenaktiven Mittel.

Es besteht weiterhin ein Bedürfnis nach einer preiswerten, gut verträglichen und stabilen Cyclosporin-Zubereitung, die insbesondere leicht herstellbar ist, die leicht mit Wasser mischbar ist und darin eine stabile Cyclosporin-Lösung bildet, die bei oraler Applikation eine gute Resorption des Cyclosporins gewährleistet und die Cyclosporin in hoher Konzentration enthalten kann.

Ein Aufgabe der vorliegenden Erfindung besteht somit darin, eine Cyclosporin-Zubereitung zur Verfügung zu stellen, die die vorstehend genannten Vorteile aufweist.

Es wurde nun überraschend gefunden, daß eine Lösung von Cyclosporin in ausschließlich wassermischbaren Hilfsstoffen nur in Kombination mit Dexpanthenol, einem anionischen Tensid und einem nichtionischen Tensid oder einer Mischung nichtionischer Tenside in Wasser stabile kolloidale Lösungen bildet, die beliebig mit Wasser verdünnbar sind, ohne daß das Cyclosporin präzipitiert. Bioverfügbarkeitsuntersuchungen zeigten eine gute Absorption des Wirkstoffes nach oraler Gabe.

Die erfindungsgemäße Cyclosporin-Lösung kann eine höhere Wirkstoffmenge je ml Lösung aufnehmen, als dies von Cyclosporin-Formulierungen im Stand der Technik bekannt ist.

Die vorliegende Erfindung betrifft somit eine Cyclosporin-Lösung, umfassend Dexpanthenol, ein anionisches Tensid und ein nichtionisches Tensid oder eine Mischung nichtionischer Tenside.

Dexpanthenol ist die Kurzbezeichnung für D-(+)-2,4-Dihydroxy-N-(3-hydroxypropyl)-3,3-dimethylbutyramid.

Das bevorzugte Cyclosporin ist Cyclosporin A.

Die erfindungsgemäße Cyclosporin-Lösung kann den Wirkstoff sowie Dexpanthenol, das anionische Tensid und das nichtionische Tensid sowie gegebenenfalls weitere pharmazeutisch verträgliche Hilfsstoffe in beliebigen Mengen enthalten, sofern die Menge von Dexpanthenol, dem anionischen Tensid und dem nichtionischen Tensid ausreicht, um eine stabile Cyclosporin-Lösung zu bilden.

Bevorzugt umfaßt die Lösung pro Gewichtsteil Cyclosporin 0,2-2 Gewichtsteile Dexpanthenol, 0,2-1 Gewichtsteil anionisches Tensid und 0,5-6 Gewichtsteile nichtionisches Tensid oder einer Mischung nichtionischer Tenside.

Im allgemeinen umfaßt die erfindungsgemäße Cyclosporin-Lösung pro Gewichtsteil Cyclosporin 0,2-2, vorzugsweise 0,5-2, beispielsweise 0,7-1,3 Gewichtsteile Dexpanthenol, 0,2-1, vorzugsweise 0,3-0,7 Gewichtsteile anionisches Tensid und 0,5-6, vorzugsweise 3-5 Gewichtsteile nichtionisches Tensid oder eines Gemisches nichtionischer Tenside.

Vorteilhaft kann die erfindungsgemäße Cyclosporin-Lösung zusätzlich ein Verdünnungsmittel umfassen. Durch das Verdünnungsmittel wird die Viskosität der Lösung reduziert. Dies hatte den Vorteil, daß bei Abfüllung der Lösung beispielsweise in Weichgelantinekapseln nach der Einnahme der Kapsel deren Inhalt sehr schnell aus der sich öffnenden Kapsel austritt und somit eine gute Resorption des Wirkstoffs gewährleistet ist.

Bei einer Trinklösung, die vor der Applikation in Wasser verdünnt wird, so daß deren Viskosität sehr stark reduziert wird, kann auf einen Verdünnungsmittelzusatz verzichtet werden.

Wenn die erfindungsgemäße Lösung ein Verdünnungsmittel enthalten soll, beträgt dessen Gehalt vorteilhaft 10-40 Gew.-%, insbesondere etwa 20 Gew.-%, bezogen auf das Gesamtgewicht der Lösung. Das bevorzugte Verdünnungsmittel ist Ethanol.

Als anionisches Tensid kann jedes herkömmliche, pharmazeutisch verträgliche anionische Tensid für die erfindungsgemäße Lösung verwendet werden. Auch kann sowohl ein anionisches Tensid alleine oder eine Mischung aus zwei oder mehr anionischen Tensiden verwendet werden. Beispiele für erfindungsgemäß verwendbare anionische Tenside sind Alkylethersulfate und Alkansulfonate. Das bevorzugte anionische Tensid ist Natriumlaurylsulfat.

Als nichtionisches Tensid kann jedes herkömmliche, pharmazeutisch verträgliche nichtionische Tensid für die erfindungsgemäße Lösung verwendet werden. Auch kann sowohl ein nichtionisches Tensid alleine oder in Mischung mit anderen nichtionischen Tensiden verwendet werden, wobei eine Mischung nichtionischer Tenside bevorzugt wird. Beispiele für erfindungsgemäß verwendbare nichtionische Tenside sind Glycerin-Polyethylenglycoloxystearat (z.B. Cremophor RH 40), ethoxyliertes hydriertes Ricinusöl und Polysorbat 80, ein Polyoxyethylen (80) sorbitanmonooleat, das unter dem Handelsnamen Tween 80 erhältlich ist. Die bevorzugten nichtionischen Tenside sind Polysorbat 80 und Glycerin-Polyethylenglycoloxystearat.

Eine bevorzugte erfindungsgemäße Lösung besteht aus etwa 11 Gew.-% Cyclosporin A, etwa 11 Gew.-% Dexpanthenol, etwa 5,6 Gew.-% anionischem Tensid, etwa 55,6 Gew.-% einer Mischung nichtionischer Tenside, und etwa 16,8 Gew.-% Verdünnungsmittel, insbesondere Ethanol. Diese Lösung eignet sich besonders zum Abfüllen in Weichgelantinekapseln, da sie aufgrund ihrer niedrigen Viskosität sehr schnell aus der sich öffnenden Kapsel austritt und eine gute Resorption des Wirkstoffs gewährleistet. Eine andere bevorzugte erfindungsgemäße Lösung besteht aus etwa 19-26 Gew.-% Cyclosporin A, etwa 8-10 Gew.-% Dexpanthenol, etwa 8-10 Gew.-.% anionischem Tensid, etwa 44-50 Gew.-% nichtionischem Tensid und etwa 12-14 Gew.-% eines Verdünnungsmittels.

Durch die Kombination von Dexpanthenol, einem anionischen Tensid und einem nichtionischen Tensid als Lösungsmittel für Cyclosporin wird eine Cyclosporin-Lösung zur Verfügung gestellt, die sich leicht mit Wasser mischt und dabei eine wäßrige, stabile, kolloidale Lösung bildet, die beliebig mit Wasser verdünnbar ist, ohne daß Cyclosporin präzipitiert. Die erfindungsgemäße Lösung stellt keine Mikroemulsion oder Mikroemulsionskonzentrat dar und besteht ausschließlich aus bekannten pharmazeutischen Stoffen. Sie kann sowohl in Kapseln abgefüllt als auch in Form einer gut schmeckenden Trinklösung dem Patienten verabreicht werden.

Im Vergleich zum Stand der Technik konnte durch die Kombination der genannten Stoffe auf eine lipophile Komponente, die zur Bildung einer Mikroemulsion notwendig ist, verzichtet werden. Völlig unerwartet übernimmt hier Dexpanthenol, obwohl es kein Tensid ist, die Rolle eines Lösungsvermittlers, der eine stabile kolloidale Lösung des Cyclosporins im Auflösungsmedium erst ergibt. Weder die in der Formulierung enthaltenen anionischen und nichtionischen Tenside alleine noch in ihrer Kombination sind in der Lage, das Cyclosporin ohne Präzipitation zu lösen.

Durch die überraschend guten Lösungseigenschaften des Dexpanthenol kann die Cyclosporin-Konzentration der erfindungsgemäßen Lösung im Vergleich zum Stand der Technik erhöht werden, so daß beispielsweise in Arzneimitteln eine erhöhte Wirkstoffkonzentration erreicht werden kann oder die zu verabreichende Menge der Lösung verringert werden kann. Damit können beispielsweise kleinere, für den Patienten leichter einzunehmende Kapseln hergestellt werden.

Die vorliegende Erfindung betrifft somit auch ein orales Arzneimittel, das eine vorstehend beschriebene Cyclosporin-Lösung umfaßt.

Bevorzugt handelt es sich bei einem solchen Arzneimittel um Kapseln, in die die Lösung abgefüllt ist. Besonders bevorzugt sind Weichgelantinekapseln. Bei Überprüfung der Auflösungsgeschwindigkeit in Medien verschiedener pH-Werte, wie sie für den Magen-Darm-Trakt typisch sind, wurde eine weitgehend pH-unabhängige Wirkstofffreisetzung aus den Kapseln gefunden.

In einer anderen Ausführungsform liegt das die erfindungsgemäße Lösung umfassende Arzneimittel als Trinklösung vor, die neben der erfindungsgemäßen Cyclosporin-Lösung weitere übliche, pharmazeutisch verträgliche Zusatzstoffe sowie beispielsweise Geschmacks- und Farbstoffe enthalten kann und die vor ihrer Einnahme beispielsweise mit Wasser auf die gewünschte Konzentration verdünnt werden kann. Die erfindungsgemäße Cyclosporin-Lösung eignet sich somit auch zur leichten Herstellung einer stabilen, wäßrigen, gut schmeckenden Trinklösung, die dem Patienten leicht verabreicht werden kann.

Nach Applikation eines erfindungsgemäßen Arzneimittels werden sehr schnell und zuverlässig die notwendigen Cyclosporin-Blutspiegel erreicht, wobei die Gleichmäßigkeit der Blutspiegel höher ist als nach Applikation des im Handel erhältlichen Präparats Neoral®.

Die beschriebene Lösung kann in Form einer verdünnten, wäßrigen Lösung zum Einnehmen oder als einzeln dosierte Arzneiform, z.B. in der Ausführung als Kapsel appliziert werden. Beispielsweise kann eine Kapsel eine Einzeldosis von 100 mg Cyclosporin enthalten.

Bei einer bevorzugten Ausführungsform des erfindungsgemäßen Arzneimittels handelt es sich demnach um Weichgelantinekapseln, die jeweils eine erfindungsgemäße Lösung aus etwa 100 mg Cyclosporin A, etwa 100 mg Dexpanthenol, etwa 50 mg Natriumlaurylsulfat, etwa 100 mg Polysorbat 80, etwa 400 mg Glycerin-Polyethylenglyceroloxystearat und etwa 150 mg Ethanol enthalten.

Das erfindungsgemäße Arzneimittel eignet sich insbesondere zur Immunsupprimierung.

Die folgenden Beispiele sollen die vorliegende Erfindung näher erläutern.

### Beispiel 1

Dieses Beispiel zeigt die Herstellung einer erfindungsgemäßen Cyclosporin-Lösung und eines erfindungsgemäßen Arzneimittels in Form von Weichgelantinekapseln.

Es wurden Weichgelantinekapseln mit einer Füllung folgender Zusammensetzung hergestellt:

| | |
|---|---|
| Cyclosporin A | 100 mg |
| Dexpanthenol | 100 mg |
| Natriumlaurylsulfat (anionisches Tensid) | 50 mg |
| Polysorbat 80 (nichtionisches Tensid) | 100 mg |
| Glycerin-Polyethylenglycoloxystearat (nichtionisches Tensid) | 400 mg |
| Ethanol (Verdünnungsmittel) | 150 mg |

Das Cyclosporin A wurde in Ethanol gelöst. Separat davon wurden Natriumlaurylsulfat, Dexpanthenol, Polysorbat 80 und Glycerin-Polyethylenglycoloxystearat unter leichtem Erwärmen klar gelöst. Beide Lösungen wurden homogen gemischt und anschließend in Weichgelantinekapseln abgefüllt.

### Beispiel 2

Mit den in Beispiel 1 hergestellten Kapseln wurde eine Absorptionsstudie an sechs Beagle Hunden durchgeführt. Je Hund wurde eine Kapsel à 100 mg Cyclosporin A im cross-over Versuch im Vergleich zu Neoral® (Zusammensetzung: Cyclosporin A, Ethanol, Glycerin, Maisöl-mono-di-tri-glyceride, Propylenglycol, Macrogol-Glycerolhydroxystearat, alpha-Tocopherol) appliziert und nach 0,5, 1,0, 1,5 und 2,0 Stunden wurden Blutproben entnommen. Die Cyclosporin A-Blutspiegel der entnommenen Proben wurden mittels eines handelsüblichen Enzymimmunoassays bestimmt. In der folgenden Tabelle sind jeweils die sich aus den Blutspiegelkurven ergebenden Mittelwerte mit den Standardabweichungen angegeben.

**Tabelle**

| **Präparat** | **Blutspiegel ng/ml** | **Standardabweichung ng/ml** |
|---|---|---|
| Neoral | | |
| 0,5 h | 457,92 | 337,28 |
| 1,0 h | 1222,83 | 406,48 |
| 1,5 h | 1616,67 | 393,71 |
| 2,0 h | 1432,33 | 243,08 |

| **Testformulierung** | | |
|---|---|---|
| 0,5 h | 435,67 | 332,11 |
| 1,0 h | 1201,5 | 328,79 |
| 1,5 h | 1398,17 | 239,36 |
| 2,0 h | 1170,67 | 111,88 |

Das Beispiel zeigt, daß nach der Applikation der erfindungsgemäßen Cyclosporin-Lösung in Form einer Kapsel sehr schnell und zuverlässig die notwendigen Blutspiegel erreicht werden, wobei die Gleichmäßigkeit der Blutspiegel höher als nach Applikation des Vergleichspräparats ist.

### Beispiel 3

Es wurde eine Cyclosporin-Lösung folgender Zusammensetzung hergestellt:

| **Bestandteile** | |
|---|---|
| Ciclosporin A | 175 mg (ca. 19,5 %) |
| Dexpanthenol | 80 mg (ca. 8,9 %) |
| Natriumlaurylsulfat (anionisches Tensid) | 80 mg (ca. 8,9 %) |
| Polysorbat 80 Glycerin-Polyethylen- | - |
| glycol-stearat | 445 mg (ca. 49,4 %) |
| Verdünnungsmittel | 120 mg (ca. 13.3 %) |
| gesamt | 900 mg |

### Beispiel 4

Es wurde eine Cyclosporin-Lösung folgender Zusammensetzung hergestellt:

| **Bestandteile** | |
|---|---|
| Ciclosporin A | 228 mg (ca. 25,3 %) |
| Dexpanthenol Natriumlaurylsulfat | 75 mg (ca. 8,4 %) |
| (anionisches Tensid) | 75 mg (ca. 8,4 %) |
| Polysorbat 80 Glycerin-Polyethylen- | - |
| glycol-stearat | 410 mg (ca. 45,5 %) |
| Verdünnungsmittel | 112 mg (ca. 12.4 %) |
| gesamt | 900 mg |

## Patentansprüche

1. Cyclosporin-Lösung, umfassend Dexpanthenol, ein anionisches Tensid und ein nichtionisches Tensid oder eine Mischung nichtionischer Tenside.

2. Cyclosporin-Lösung nach Anspruch 1, worin das Cyclosporin Cyclosporin A ist.

3. Cyclosporin-Lösung nach einem der vorhergehenden Ansprüche, wobei die Lösung pro Gewichtsteil Cyclosporin 0,2-2 Gewichtsteile Dexpanthenol, 0,2-1 Gewichtsteil anionisches Tensid und 0,5-6 Gewichtsteile nichtionisches Tensid oder einer Mischung nichtionischer Tenside umfaßt.

4. Cyclosporin-Lösung nach einem der vorhergehenden Ansprüche, die zusätzlich ein Verdünnungsmittel umfaßt.

5. Cyclosporin-Lösung nach Anspruch 4, worin der Verdünnungsmittelgehalt 10-40 Gew.-% bezogen auf das Gesamtgewicht der Lösung beträgt.

6. Cyclosporin-Lösung nach Anspruch 4 oder 5, worin das Verdünnungmittel Ethanol ist.

7. Cyclosporin-Lösung nach einem der vorhergehenden Ansprüche, worin das anionische Tensid Natriumlaurylsulfat ist.

8. Cyclosporin-Lösung nach einem der vorhergehenden Ansprüche, worin die nichtionischen Tenside Polysorbat 80 und Glycerin-Polyethylenglycoloxystearat sind.

9. Cyclosporin-Lösung nach einem der Ansprüche 4-8, bestehend aus etwa 11 Gew.-% "Cyclosporin A, etwa 11 Gew.-% Dexpanthenol, etwa 5,6 Gew.-% anionischem Tensid, etwa 55,6 Gew.-% einer Mischung nichtionischer Tenside und etwa 16,8 Gew.-% eines Verdünnungsmittels, insbesondere Ethanol.

10. Cyclosporin-Lösung nach einem der Ansprüche 4-8, bestehend aus etwa 19-26 Gew.-% Cyclosporin A, etwa 8-10 Gew.-% Dexpanthenol, etwa 8-10 Gew.-% anionischem Tensid, etwa 44-50 Gew.-% nichtionischem Tensid und etwa 12-14 Gew.-% eines Verdünnungsmittels.

11. Orales Arzneimittel, umfassend eine Lösung nach einem der Ansprüche 1-10.

12. Arzneimittel nach Anspruch 11, wobei die Lösung in Kapseln abgefüllt ist.

13. Arzneimittel nach Anspruch 12, wobei die Kapseln Weichgelantinekapseln sind.

14. Arzneimittel nach Anspruch 11, wobei die Lösung in Form einer Trinklösung vorliegt.

15. Verwendung einer Lösung nach einem der Ansprüche 1-10 zur Herstellung einer stabilen, wäßrigen, kolloidalen Cyclosporin-Lösung.

16. Verwendung einer Lösung nach einem der Ansprüche 1-10 zur Herstellung eines oralen Arzneimittels zur Immunsupprimierung.

## Claims

1. Cyclosporin solution comprising dexpanthenol, an anionic surfactant and a nonionic surfactant or a mixture of nonionic surfactants.

2. Cyclosporin solution according to Claim 1, in which the cyclosporin is cyclosporin A.

3. Cyclosporin solution according to either of the preceding claims, where the solution comprises 0.2-2 parts by weight of dexpanthenol, 0.2-1 part by weight of anionic surfactant and 0.5-6 parts by weight of nonionic surfactant or a mixture of nonionic surfactants per part by weight of cyclosporin.

4. Cyclosporin solution according to any of the preceding claims, which additionally comprises a diluent.

5. Cyclosporin solution according to Claim 4, in which the diluent content is 10-40% by weight based on the total weight of the solution.

6. Cyclosporin solution according to Claim 4 or 5, in which the diluent is ethanol.

7. Cyclosporin solution according to any of the preceding claims, in which the anionic surfactant is sodium lauryl sulphate.

8. Cyclosporin solution according to any of the preceding claims, in which the nonionic surfactants are polysorbate 80 and glycerol-polyethylene glycol oxystearate.

9. Cyclosporin solution according to any of Claims 4-8, consisting of about 11% by weight of cyclosporin A, about 11% by weight of dexpanthenol, about 5.6% by weight of anionic surfactant, about 55.6% by weight of a mixture of nonionic surfactants and about 16.8% by weight of a diluent, in particular ethanol.

10. Cyclosporin solution according to any of Claims 4-8, consisting of about 19-26% by weight of cyclosporin A, about 8-10% by weight of dexpanthenol, about 8-10% by weight of anionic surfactant, about 44-50% by weight of nonionic surfactant and about 12-14% by weight of a diluent.

11. Oral pharmaceutical comprising a solution according to any of Claims 1-10.

12. Pharmaceutical according to Claim 11, where the solution is used to fill capsules.

13. Pharmaceutical according to Claim 12, where the capsules are soft gelatin capsules.

14. Pharmaceutical according to Claim 11, where the solution is in the form of an oral solution.

15. Use of a solution according to any of Claims 1-10 for producing a stable aqueous colloidal cyclosporin solution.

16. Use of a solution according to any of Claims 1-10 for producing an oral pharmaceutical for immunosuppression.

## Revendications

1. Solution de cyclosporine, comprenant du dexpanthénol, un agent tensioactif anionique et un agent tensioactif non ionique ou un mélange d'agents tensioactifs non ioniques.

2. Solution de cyclosporine suivant la revendication 1, dans laquelle la cyclosporine est de la cyclosporine A.

3. Solution de cyclosporine suivant l'une des revendications précédentes, dans laquelle la solution comprend, par partie en poids de cyclosporine, 0,2-2 parties en poids de dexpanthénol, 0,2-1 partie en poids d'agent tensioactif anionique et 0,5-6 parties en poids d'agent tensioactif non ionique ou d'un mélange d'agents tensioactifs non ioniques.

4. Solution de cyclosporine suivant l'une des revendications précédentes, qui comporte, en outre, un diluant.

5. Solution de cyclosporine suivant la revendication 4, dans laquelle la teneur en diluant est de 10-40% en poids par rapport au poids total de la solution.

6. Solution de cyclosporine suivant l'une des revendications 4 et 5, dans laquelle le diluant est de l'éthanol.

7. Solution de cyclosporine suivant l'une des revendications précédentes, dans laquelle l'agent tensioactif anionique est du laurylsulfate de sodium.

8. Solution de cyclosporine suivant l'une des revendications précédentes, dans laquelle les agents tensioactifs non ioniques sont du Polysorbat 80 et de l'oxystéarate de glycérine-polyéthylèneglycol.

9. Solution de cyclosporine suivant l'une des revendications 4-8, constituée d'environ 11% en poids de cyclosporine A, d'environ 11% en poids de dexpanthénol, d'environ 5,6% en poids d'agent tensioactif anionique, d'environ 55,6% en poids d'un mélange d'agents tensioactifs non ioniques et d'environ 16,8% en poids d'un diluant, en particulier de l'éthanol.

10. Solution de cyclosporine suivant une des revendications 4-8, constituée d'environ 19-26% en poids de cyclosporine A, d'environ 8-10% en poids de dexpanthénol, d'environ 8-10% en poids d'agent tensioactif anionique, d'environ 44-50% en poids d'agent tensioactif non ionique et d'environ 12-14% en poids d'un diluant.

11. Médicament oral, comportant une solution suivant l'une des revendications 1 à 10.

12. Médicament suivant la revendication 11, dans lequel la solution est transvasée dans des capsules.

13. Médicament suivant la revendication 12, dans lequel les capsules sont des capsules de gélatine molle.

14. Médicament suivant la revendication 11, dans lequel la solution se présente sous la forme d'une solution à boire.

15. Utilisation d'une solution suivant l'une des revendications 1-10 pour la préparation d'une solution de cyclosporine colloïdale, aqueuse, stable.

16. Utilisation d'une solution suivant une des revendications 1-10 pour la préparation d'un médicament oral destiné à l'immuno-suppression.
